(19) Europäisches Patentamt
European Patent Office
Office européen des brevets

(11) Veröffentlichungsnummer : **0 160 824 B2**

(12) **NEUE EUROPÄISCHE PATENTSCHRIFT**

(45) Veröffentlichungstag der neuen Patentschrift : **03.08.94 Patentblatt 94/31**

(51) Int. Cl.$^5$ : **C08F 299/02,** C08F 236/02, C09D 4/00

(21) Anmeldenummer : **85103655.8**

(22) Anmeldetag : **27.03.85**

# (54) Umsetzungsprodukt von olefinisch ungesättigten Verbindungen mit wasserstoffaktiven Verbindungen, Verfahren zu dessen Herstellung und darauf basierende 2-Komponentenlacke.

(30) Priorität : **04.04.84 DE 3412658**
**08.03.85 DE 3508399**

(43) Veröffentlichungstag der Anmeldung : **13.11.85 Patentblatt 85/46**

(45) Bekanntmachung des Hinweises auf die Patenterteilung : **06.09.89 Patentblatt 89/36**

(45) Bekanntmachung des Hinweises auf die Entscheidung über den Einspruch : **03.08.94 Patentblatt 94/31**

(84) Benannte Vertragsstaaten : **AT BE CH DE FR GB IT LI NL SE**

(56) Entgegenhaltungen :
**EP-A- 0 161 697**
**DE-B- 835 809**
**GB-A- 2 048 913**
**US-A- 2 759 913**
**US-A- 4 373 608**

(56) Entgegenhaltungen :
**US-A- 4 408 018**
**Tetrahedron Letters no. 37, 1973, pp. 3597-3600**
**House, H.O. "Modern Synthetic Reactions", 2nd Ed., 1972, pp. 595-623**

(73) Patentinhaber : **HOECHST AKTIENGESELLSCHAFT**
**D-65926 Frankfurt (DE)**

(72) Erfinder : **Brindöpke, Gerhard, Dr.**
**Loreleistrasse 18**
**D-6230 Frankfurt am Main 80 (DE)**
Erfinder : **Walz, Gerd, Dr.**
**Pfingstbornstrasse 99**
**D-6200 Wiesbaden (DE)**
Erfinder : **Waldmann, Karl, Dr.**
**Am Rehsteig 25**
**D-6232 Bad Soden am Taunus (DE)**
Erfinder : **Schön, Manfred, Dr.**
**Saalburgring 3**
**D-6054 Rodgau (DE)**
Erfinder : **Kleiner, Hans-Jerg, Dr. Ing.**
**Altkönigstrasse 11a**
**D-6242 Kronberg/Taunus (DE)**

EP 0 160 824 B2

Jouve, 18, rue Saint-Denis, 75001 PARIS

## Beschreibung

Es ist bekannt, ungesättigte Verbindungen, wie Zimtsäureester, mit H-aktiven Verbindungen, z.B. Malonsäureester oder Acetessigester, durch Michael-Addition unter Bildung von substituierten Verbindungen, z.B. substituierten Malonsäureestern, umzusetzen (Krauch-Kunz « Namensreaktionen der organischen Chemie » 5. Auflage 1976, Seite 42).

Ferner ist es bekannt, ein OH-Gruppen enthaltendes Acrylatharz bzw. ein mit ε-Caprolacton modifiziertes Acrylatharz im Gemisch mit Polyisocyanaten als Zweikomponentenlacke zu verwenden (DE-PS 3 005 945, 3 027 776 und 3 148 022). Eine weitere Druckschrift beschreibt die Umsetzung von Epoxidgruppen enthaltenden Acrylatcopolymerisaten mit einem teilblockierten Isocyanat und die Verwendung des Reaktionsproduktes als Lackbindemittel (DE-OS 3 130 545).

Die bekannten Produkte haben sich zum Teil gut bewährt. Es wurde jedoch schon versucht, umweltfreundlichere Produkte herzustellen, indem man von Systemen ohne freies Isocyanat ausgeht.

So ist in einer anderen Druckschrift ein Oxazolidin enthaltendes Acrylatharz beschrieben, das Wasser oder Luftfeuchtigkeit als Härter nutzt (EP-OS 34 720). Dieses System hat den Nachteil, daß die gehärtete Oberfläche einem tieferen Eindringen von Wasser in die unteren Schichten des Überzugs entgegenwirkt und so eine vollständige Härtung durch die ganze Schichtdicke verhindert.

Es ist auch ein ohne Isocyanat reagierendes Zweikomponenten-System bekannt. Dieses besteht aus einem Epoxidgruppen enthaltenden Acrylharz, das mit einem anderen Acrylharz, das tertiäre Aminogruppen enthält, härtbar ist. Bei dem nach diesem Verfahren hergestellten Produkt führt der zu geringe Vernetzungsgrad zu einer ungenügenden chemischen Beständigkeit, so daß die mit diesem System hergestellten Überzüge nur für ein beschränktes Anwendungsgebiet geeignet sind.

In der US-PS 4 408 018 werden Acrylpolymere beschrieben, in die Acetoacetat-Gruppierungen eingeführt worden sind und die über die Michael-Addition in Gegenwart starker Basen mit α,β-olefinisch ungesättigten Estern vernetzt werden können. Die Einführung der Acetoacetat-Gruppe erfolgt über die Acetessigester der Hydroxymethylacrylate oder -methacrylate und nachfolgende Copolymerisation mit weiteren copolymerisierbaren Monomeren oder durch Reaktion OH-gruppenhaltiger Polymere mit dem Vorprodukt der Acetessigesterkomponente, dem Diketen. Als organische Komponente, die mit den Acetoacetatgruppen enthaltenen Polymeren vernetzt werden können, sind Polyacrylate mit mehr als zwei Acrylatgruppen, Reaktionsprodukte von Polyisocyanaten mit Hydroxylgruppen enthaltenden Acrylsäureestern und Reaktionsprodukte von Epoxidharzen mit Acrylsäure genannt. Diese Verbindungen haben aber den Nachteil, daß die als Katalysatoren wirkenden starken Basen wie Alkalihydroxide oder -alkoholate zu einer starken Vergilbung und Trübung des Lackes führen.

Der Erfindung liegt nunmehr die Aufgabe zugrunde, das Angebot der auf dem Markt befindlichen Lackbindemittel durch ein System zu erweitern, das zur Härtung keine freien Isocyanate benötigt, also umweltfreundlich ist. Hierbei werden Katalysatoren eingesetzt, die die Topfzeit nicht verkürzen und auch keine Vergilbung der Lackfilme bewirken und die bei Verwendung geringer Katalysatormengen Überzüge ergeben, die hohen Anforderungen hinsichtlich Härtungseigenschaften und chemischer Beständigkeit entsprechen.

Die vorliegende Erfindung betrifft daher einen

1. 2-Komponentenlack bestehend aus

A) Verbindungen mit mindestens zwei $R^1R^2C = CR^3$-X-Gruppen (I),

B) Verbindungen, die

a) mindestens zwei aktive H-Atome oder

b) mindestens zwei Gruppen mit aktiven H-Atomen des Typs -AH- (II) oder

c) mindestens ein aktives H-Atom und mindestens eine Gruppe des Typs (II) enthalten bzw. die entsprechende Anzahl dieser Gruppe (II) bilden,

worin in Formel (I) bedeuten

X = -CO-, das entweder direkt oder über den Rest eines mehrwertigen Alkohols oder eines Amins an eine weitere $R^1R^2C = CR^3$-Gruppe gebunden ist,

$R^1$ = Wasserstoff oder ein Kohlenwasserstoff-Rest mit 1 bis 10 C-Atomen,

$R^2$ = Wasserstoff, ein Kohlenwasserstoff-Rest mit 1 bis 10 C-Atomen, eine Estergruppe mit dem Rest $R^{14}$ eines einwertigen Alkohols mit bis zu 12 C-Atomen, -CN, $-NO_2$, eine CO-$NHR^1$- oder eine CO-$R^1$- Gruppe,

$R^3$ = $R^2$ und hiermit gleich oder verschieden ist, und in Formel (II)

-AH- für eine der Gruppierungen

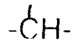

oder -SH- steht,

C) einem Katalysator aus der Gruppe Halogenide von quartären Ammoniumverbindungen, allein oder im Gemisch mit Kieselsäurealkylestern, oder organischen Phosphoniumsalzen mit 1 bis 20 C-Atomen im Alkylrest und/oder Arylrest sowie Phosphanen, für sich oder in Kombination mit üblichen Zusatzstoffen.

In dem 2-Komponentenlack ist Komponente A) ein Michael-Acceptor, Komponente B) ein Michael-Donator.

Dieser 2-Komponentenlack hat den Vorteil, daß es aus Komponenten hergestellt wird, die keine toxischen Bestandteile enthalten und die daher ohne besondere Vorsichtsmaßnahmen angewendet werden können.

Obwohl bei der Umsetzung von Verbindungen A) mit jeweils zwei Gruppen (I) mit Verbindungen B) mit zwei aktiven H-Atomen bzw. zwei Gruppen (II), auch wenn diese nur ein H-Atom enthalten, aufgrund der beiderseitigen Bifunktionalität zu erwarten ist, daß hierbei ausschließlich eine Kettenverlängerung eintritt, erhält man überraschenderweise die erfindungsgemäßen gehärteten vernetzten Umsetzungsprodukte. Die wirksamen Gruppen der Verbindungen A) und B) können auch in einem einzigen Molekül enthalten sein, so daß Systeme vorliegen, die durch intermolekulare Vernetzung härtbar und selbstvernetzend sind.

Wenn eine höhere Reaktivität und damit stärkere Vernetzung des Produktes angestrebt wird, kann man mit Vorteil so vorgehen, daß in mindestens einer der Verbindungen A) und B) drei oder mehr Gruppen des Typs (I) bzw. aktive H-Atome und/oder Gruppierungen des Typs (II) vorhanden sind.

Nach einer Ausführungsform der Erfindung kann der Rest $R^1R^2C = CR^3$-X (I) vn einer ein- oder mehrfach ungesättigten höchsten zweibasischen Carbonsäure, z.B. Mono- und/oder Dicarbonsäure, mit 2 bis 10, vorzugsweise 3 bis 6 C-Atomen, wie Zimtsäure, Crotonsäure, Citraconsäure oder deren Anhydrid, Mesaconsäure, Fumarsäure, Dehydrolävulinsäure, Sorbinsäure, vorzugsweise jedoch Acrylsäure, Methacrylsäure und/oder Maleinsäure oder deren Anhydrid abgeleitet sein, ferner auch von ungesättigten Ketonen, wie Divinylketon, Dibenzalaceton; ferner von ungesättigten Nitrilen, wie Maleinsäuremononitril-monoestern mehrwertiger Alkohole, von Cyanacrylsäureestern der Formel $H_2C = C(CN)-COOR$, von Nitriten der Formel $ROOC-R^3C = CH-NO_2$, von Alkylidenmalonsäureestern der Formel $ROOC-C-(COOR^4) = CR^1R^2$, Alkylidenacetessigestern der Formel $ROOC-C(CO-CH_3) = CR^1R^2$ bzw. den entsprechenden Nitrilen, wobei in vorstehenden Formeln R der Rest eines mehrwertigen Alkohols, $R^1$ und $R^2$ Wasserstoff oder Alkyl ist, oder dergleichen. Die Gruppen (I) liegen in der Regel in Form von Estern oder Amiden gebunden vor. Sie können an den Rest eines mehrwertigen Alkohols, einer Verbindung mit NH-Gruppen wie eines Polyamins, Polyamids, Polyiminoamids oder eines mehrwertigen Phenols, vorzugsweise eines Oligomeren oder Polymeren gebunden sein. So kann sich die Verbindung A) beispielsweise ableiten von gesättigten und/oder ungesättigten, OH-Gruppen enthaltenden Polyäthern oder Polyestern, z.B. solchen auf der Basis von Maleinsäure, Phthalsäure und Diolen; OH-Gruppen enthaltenden Acrylharzen; aliphatischen oder vorzugsweise aromatischen, gegebenenfalls OH-Gruppen enthaltenden Epoxidharzen, z.B. solchen auf der Basis von Diphenylolpropan und/oder -methan, Hydantoin und-/oder Aminharzen. Dabei kann der esterartig gebundene Rest (I) z.B. durch Anlagerung von Acryl- oder Methacrylsäure an die Epoxidgruppe entstanden sein. Als Ausgangssubstanzen für A) geeignete mehrwertige Alkohole sind beispielsweise Alkandiole und -triole, wie Äthandiol, die verschiedenen Propan-, Butan-, Hexan-, Octandiole oder deren Homologe, die entsprechenden oligomeren Äther, ferner Glycerin, Trimethyloläthan oder -propan, Hexantriol, Pentaerythrit, Dipentaerythrit, Sorbit, Polyvinylalkohol oder dergleichen.

Als NH-Gruppen enthaltende Ausgangsverbindungen für die Verbindungen A werden z.B. genannt Alkylendiamine und deren Oligomere, wie Äthylendiamin, Propylendiamin, Butylendiamin, Diäthylentriamin, Tetramine und höhere Homologe dieser Amine, ferner Aminoalkohole, wie Diäthanolamin oder dergleichen. Als Amine kommen z.B. auch Aminocarbonsäureester mehrwertiger Alkohole in Frage. Als Verbindungen mit NH-Gruppen kommen z.B. in Frage Acryl- oder Methacrylsäure-polyamide, ferner Polyurethane, z.B. Polyisocyanate, die in Form von Polyurethangruppen verkappt sind wie solche, die durch Umsetzung von Hydroxyäthylacrylat an Polyisocyanate erhalten werden, Aminharze wie Methylolmelamine, vorzugsweise Hexamethylolmelamin, Harnstoffharze, wobei der Rest (I) mit der Gruppierung -CO- an die Amingruppen dieser Verbindungen als Amid gebunden ist. Falls diese Aminverbindungen über OH-Gruppen bzw. Alkylolgruppen verfügen, ist es auch möglich, daß der Rest (I) unmittelbar über eine Estergruppe (Formel III s. Formelblatt) oder indirekt über eine Äthergruppe (Formel IV s. Formelblatt) an diese Harze gebunden ist. Für die Ätherbindung des Restes (I) kann man also von einem Hydroxyalkylester oder einem Hydroxyalkylamid einer ungesättigten Säure, wie Acrylsäure, ausgehen. Dasselbe gilt für eine entsprechende Bindung an Polyhydroxyverbindungen.

Die Gruppierung -AH- (II) leitet sich in der Verbindung B)

aa) für die Bedeutung

$$-\overset{|}{C}H-$$

von einer Verbindung mit der Gruppierung $-CO-CHR^1-CO-$, $NC-CHR^1-CO-$, $NC-CH_2-CN$, $= PO-CHR^1-CO-$,

= PO-$CHR^1$-CN, = PO-$CHR^1$-PO =, -CO-$CHR^1$- $NO_2$, worin $R^1$ vorzugsweise Wasserstoff ist, und

bb) für die Bedeutung -SH von einem Thioalkoholsäureester, -amid und/oder einem Mercaptan ab.

β-Dioxoverbindungen sind bevorzugt.

Geeignete Verbindungen B) vom Typ aa) sind beispielsweise Ketone, wie Acetylaceton, Benzoylaceton, Acetyldibenzoylmethan, ferner Ester der gegebenenfalls alkylsubstituierten Acetessigsäure wie α- und/oder γ-Methylacetessigsäure, oder der Acetondicarbonsäure, esterartig gebundene Malonsäureeinheiten der Malonsäure und deren Monoalkylderivate, geradkettig oder verzweigt, mit 1 bis 6 C-Atomen im Alkylrest, z.B. Methyl, Äthyl und n-Butyl oder auch Phenyl, oder der Cyanessigsäure mit ein- bis 6-wertigen Alkoholen mit 1 bis 10 C-Atomen. Die alkylsubstituierten Ester, z.B. α-Methyl- oder α,γ-Dimethylacetessigester, haben nur ein aktives H-Atom und werden daher vorzugsweise in Form von Di- oder Polyestern mehrwertiger Alkohole eingesetzt, um eine genügende Anzahl reaktiver Gruppen zur Verfügung zu haben. Geeignete Alkohole für die Veresterung vorstehender Säuren sind z.B. Methanol, Äthanol, Butanol, Octanol und/oder, was bevorzugt ist, mehrwertige Alkohole bzw. Polyhydroxyverbindungen wie die als Ausgangssubstanzen für A) genannten. Weitere Verbindungen B) sind z.B. Acetessigester, Äthandiol-bisacetessigester, Glycerintris-malonsäureester, Trimethylolpropantris-acetessigester, Teilester dieser Säuren mit mehrwertigen Alkoholen, ebenfalls die entsprechenden Ester von OH-Gruppen enthaltenden Acrylharzen, Polyestern, Polyäthern, Polyesteramiden und -imiden, Polyhydroxylaminen, ferner Nitrile dieser Säuren, sofern diese existieren, z.B. Malonsäuremono- oder -dinitril, Alkoxycarbonyl-methanphosphonsäureester und die entsprechenden Bis-methanphosphonsäureester (Formel VII s. Formelblatt). Die vorstehend genannten Säuren können auch in Form von Amiden an Amine, vorzugsweise Polyamine, gebunden sein, z.B. an die im Zusammenhang mit Verbindung A) genannten, die auch Oligomere und/oder Polymere einschließlich Aminharze umfassen, wobei aliphatische Amine bevorzugt sind.

Als Verbindungen aa) eignen sich auch reaktive Nitroverbindungen, z.B. Nitroessigsäurederivate, wie Tris-(nitroessigsäure)-glycerinester oder Trimethylolpropan-nitroessigsäureester.

Geeignete Verbindungen B) mit einer Gruppierung -SH sind z.B. Thioglykolsäure-, β-Mercaptopropionsäure- oder Thiosalicylsäureester von mehrwertigen Alkoholen und Thioalkoholäther und -ester, Mercaptane, beispielsweise Äthyl-, Propylmercaptan und deren Homologe bzw. deren Äther, z.B. Thioglycerin, Substitutionsprodukte cyclischer Amine mit Thioalkanolen oder dergleichen.

Unter Verbindungen B), die Gruppen des Typs (II) bilden, sind z.B. Diketen sowie dessen Mono-α-Alkylsubstitutionsprodukte, ferner Tetrahydrodioxin zu nennen, die mit geeigneten Komponenten unter Bildung von Acetessigester- oder -amid-Gruppen reagieren können.

Die Reaktionskomponente B) kann an mindestens eine mehrwertige Verbindung der Gruppe ein- oder mehrwertige Alkohole, OH-Gruppen enthaltende Polymere, z.B. die weiter oben genannten, Polyamine und Polymercaptane gebunden sein und ist, bezogen auf die CH-Funktion mehrwertig. So kann sie beispielsweise durch Veresterung eines Polyepoxids mit einer die Gruppierung -AH- (II) bildenden Carbonsäure, z.B. Cyanessigsäure (Gleichung V s. Formelblatt) hergestellt worden sein. Man erhält so eine Komponente B) mit zwei aktiven H-Atomen je Epoxydgruppe. Hierbei lassen sich aromatische oder aliphatische Polyepoxide einsetzen, z.B. die oben genannten.

Falls die Gruppierung -AH- die Bedeutung

$$-\overset{|}{C}H-$$

hat, kann man z.B. von 1 Mol eines Alkylendiamins ausgehen, das mit 2 Mol Acetessigester umgesetzt wird unter Bildung der Verbindung VI (S. Formelblatt), die ebenfalls vier durch Amidgruppen aktivierte H-Atome aufweist.

Als Halogenide von quartären Ammoniumverbindungen können Alkyl-, Aryl- und/oder Benzylammoniumbromide, -chloride und insbesondere -fluoride eingesetzt werden, gegebenenfalls in Kombination mit Kieselsäure-alkylestern, um die Aktivität des Katalysators noch zu verbessern. Im einzelnen seien z.B. Alkylbenzyldimethylammoniumhalogenide (Alkyl = $C_{16}$-$C_{22}$), Benzyltrimethylammoniumhalogenide, Tetrabutylammoniumhalogenide, insbesondere jeweils die Fluoride sowie einpolymerisiertes Triphenylvinylphosphoniumfluorid genannt. Weiterhin eignen sich als Katalysatoren die den vorstehenden Ammoniumhalogeniden entsprechenden organischen Phosphoniumsalze mit 1 bis 20 C-Atomen im Alkylrest und/oder Arylrest, z.B. Trimethylbenzyl-phosphoniumhalogenide, Tributylhexadecylphosphoniumbromid. Weitere geeignete Katalysatoren sind die Phosphane (früher als Phosphine bezeichnet) z.B.

a) tertiäre Phosphane der allgemeinen Formel $P(CH_2\text{-}Y)_3$, in der Y gleich oder verschieden ist und den Rest -OH, -$CH_2CN$ oder -$N(Z)_2$ bedeutet, wobei Z ein Alkylrest mit 1 bis 5 C-Atomen ist, wie Tris-2-cyanoethylphosphan, Trisdiethylaminomethylphosphan, vorzugsweise Trishydroxymethylphosphan und

Trisdimethyl-aminomethylphosphan;

b) tertiäre Phosphane der allgemeinen Formel $P(R^4,R^5,R^6)$ in der die Reste $R^4$, $R^5$ und $R^6$ einen Alkylrest mit 1 bis 12 C-Atomen oder einen Phenylrest, unsubstituiert oder substituiert mit mindestens einer Alkyl-, Alkoxy- oder Dialkylaminogruppe mit jeweils 1 bis 4 C-Atomen im Alkylrest bedeuten, $R^4$, $R^5$ und $R^6$ gleich oder verschieden sind, wobei jedoch mindestens einer der Reste einen Phenylrest darstellt, wie

Triphenylphosphan, Tris-p-tolylphosphan, Tris-o-anisylphosphan, Tris-p-dimethylaminophenylphosphan, Phenyl-di-p-anisylphosphan, Phenyldi-o-anisylphosphan, Diphenyl-p-anisylphosphan, Diphenyl-o-anisylphosphan, Diphenyl-p-dimethylaminophenylphosphan, Butyl-diphenylphosphan, Methyl-ditolylphosphan, Ethyl-di-p-anisylphosphan, (Diethylaminomethyl)-di-phenylphosphan, vorzugsweise Tris-p-anisylphosphan, Methyldiphenylphosphan und Methyl-di-p-anisylphosphan, und

c) Iminophosphorane der allgemeinen Formel $(R^7,R^8,R^9)P = N\text{-}C(R^{10}, R^{11}, R^{12})$, in der $R^7$, $R^8$, $R^9$ gleich oder verschieden sind und einen Alkylrest mit 1 bis 12 C-Atomen oder einen Phenylrest, unsubstituiert oder substituiert mit mindestens einer Alkyl-, Alkoxy- oder Dialkylaminogruppe mit jeweils 1 bis 4 C-Atomen im Alkylrest bedeuten und $R^{10}$, $R^{11}$, $R^{12}$ gleich oder verschieden sind und einen Alkylrest mit 1 bis 5 C-Atomen oder einen Phenylrest darstellt, wie $\alpha$,$\alpha$-Dimethylbenzylimino-tris(dimethylamino-)-phosphoran, $\alpha$,$\alpha$-Dimethylbenzyliminomethyl-diphenylphosphoran, t-Butylimino-tri-phenyl-phosphoran, vorzugsweise $\alpha$,$\alpha$-Dimethylbenzylimino-tri-butylphosphoran.

Die genannten Katalysatoren oder Katalysatorengemische können in Gegenwart von an sich bei Raumtemperatur nicht wirksamen tertiären aliphatischen Aminen, wie beispielsweise Triethylamin, N-Methyldiethanolamin, N-Methyl-di-isopropanolamin oder N-Butyldiethanolamin verwendet werden. Diese Hilfsstoffe können zu 0,1-5, vorzugsweise 0,1-1 Gew.-%, anwesend sein.

Die Menge des Katalysators beträgt im allgemeinen 0,01 bis 5, vorzugsweise 0,02 bis 2 Gew.-%, bezogen auf den Gesamtfeststoffgehalt des Ausgangsproduktes. Sie kann je nach der Reaktivität der Verbindungen A) und B) und der beabsichtigten Verfahrensführung variiert werden. Die Katalysatorzugabe kann auch portionsweise, d.h. in mehreren Stufen, erfolgen.

Diese Verfahren gehen sehr glatt vor sich. Da vorzugsweise von oligomeren und/oder polymeren Verbindungen A) und B) ausgegangen wird, erhält man auch oligomere und/oder polymere Reaktionsprodukte. Die Reaktion erfolgt im allgemeinen bei -10 bis 180, vorzugsweise 0 bis 100, insbesondere 20 bis 80°C. Zum Beispiel erhält man bei Raumtemperatur nach 2 bis 24 Stunden oder bei 60°C nach 10 bis 40 Minuten Produkte mit guter Härte.

Nach einer Ausführungsforderung der Erfindung werden die Verbindungen A) im Gemisch mit einem untergeordneten Anteil von solchen Verbindungen A), die nur eine Gruppe der Formel I enthalten, bzw. die Verbindungen B) im Gemisch mit einem untergeordneten Anteil von Verbindungen B), die nur ein aktives H-Atom bzw. nur eine Gruppe der Formel (II) enthalten, umgesetzt. Von dieser Möglichkeit wird man aber nur dann Gebrauch machen, wenn man die Vernetzungsdichte verringern will und die damit verbundenen Eigenschaften des Produktes entsprechend variieren möchte. Dies ist vor allem dann der Fall, wenn mindestens eine der Komponenten A) und B) durch einen verhältnismäßig hohen Anteil an reaktiven Gruppen, z.B. bei Verwendung eines monomolekularen Hexaacrylsäureesters, eine besonders starke Vernetzung bewirkt, so daß gegebenenfalls mit einer unerwünschten Verstrammung des Reaktionsprodukts und daher der Gefahr einer etwaigen Versprödung zu rechnen ist. Bei dieser Ausführungsform wird der Anteil der Verbindungen mit nur einer Gruppe (I) bzw. (II) maximal 20, vorzugsweise maximal 10, insbesondere bis zu 5 Gew.-%, bezogen auf die jeweils analoge Verbindung A) bzw. B), betragen. Mit einem solchen Zusatz kann man die Härte und Elastizität des Reaktionsproduktes in einem bestimmten Ausmaß steuern. Die für diese Variante einzusetzenden Verbindungen A) mit nur einer reaktiven Gruppe (I) sind z.B. die Ester oder Amine der für A) aufgezählten ungesättigten Carbonsäuren, die jedoch nur mit einwertigen Alkoholen oder Monoaminen verestert bzw. unter Amidbildung umgesetzt worden sind.

Geeignete Verbindungen B) für diese Ausführungsform sind z.B. solche mit den oben unter aa) genannten Gruppierungen, in denen $R^1$ eine andere Bedeutung als Wasserstoff hat, beispielsweise die Alkylsubstitutionsprodukte von Acetessigsäure und Malonsäure, die nur mit einem einwertigen Alkohol verestert oder mit einem Monoamin umgesetzt worden sind. Verbindungen B) vom Typ bb) sind Alkanolmercaptane, die nur eine Mercaptangruppe enthalten, z.B. Äthylmercaptan.

Das Verfahren läßt sich in einer oder mehreren Stufen durchführen. In der Regel wird man jedoch aus arbeitsökonomischen Gründen das Einstufenverfahren vorziehen, indem man beispielsweise mit äquivalenten Anteilen der Komponenten mit jeweils zwei aktiven Gruppen bzw. H-Atomen arbeitet (Gleichung XII s. Formelblatt s. unterstrichene H-Atome). Die Wahl der Verfahrensstufen, die Topfzeit und Eigenschaften des Produktes hängen daher von den Verfahrensbedingungen, d.h. von der Art und Menge der Ausgangsstoffe, der Dosierung des Katalysators und der Temperaturführung ab. So läßt sich die Elastizität des vernetzten Produktes innerhalb eines Toleranzbereiches z.B. durch die Kettenlänge der für A) oder B) eingesetzten Oligomeren und/oder Polymeren steuern.

Obwohl das Verfahren in der Regel diskontinuierlich betrieben wird, liegt es auch im Rahmen der Erfindung, das Vermischen der Komponenten und den Reaktionsverlauf kontinuierlich durchzuführen, beispielsweise mittels einer automatischen Lackiervorrichtung.

Bei der mehrstufigen Verfahrensführung kann man eine oder mehrere der Komponenten portionsweise zugeben. Beispielsweise kann in erster Stufe 1 Mol Butandiol-bis-acrylat mittels 2 Mol Malonsäurediamid umgesetzt werden (Gleichung XI s. Formelblatt) unter Bildung eines Produktes, das zwei Gruppen (II) mit je einem aktiven (unterstrichenen) H-Atom enthält. Dieses Produkt kann in mindestens einer weiteren Stufe mit weiteren Molekülen einer Verbindung A) umgesetzt werden unter Kettenverlängerung und Vernetzung. Auf analoge Weise läßt sich z.B. ein Mol Trimethylolpropan-tirs-acrylat oder -methacrylat in erster Stufe mit drei Mol Acetylaceton umsetzen.

Die Vernetzung in zweiter und gegebenenfalls noch folgenden Stufen kann beispielsweise durch Katalysatorzugabe erfolgen. Man kann dabei so vorgehen, daß man in erster Stufe nur mit einer verhältnismäßig geringen Katalysatormenge, z.B. weniger als 2%, oder mit einem verhältnismäßig wenig aktiven Katalysator arbeitet. Falls die zweite oder folgende Stufen relativ kurz nach der Herstellung des Vorproduktes durchgeführt werden, ist es nicht erforderlich, den etwa in erster Stufe eingesetzten Katalysator zu entfernen. Man kann nun in zweiter Stufe entweder eine höhere Dosis desselben Katalysators und/oder einen anderen Katalysator verwenden. Es besteht auch die Möglichkeit, in erster Stufe ein Vorprodukt herzustellen, das über eine längere Zeitspanne lagerstabil ist und sogar zum Verarbeiter verschickt werden kann. In diesem Fall wird man den Katalysator mit Vorteil, z.B. durch Neutralisation oder Abdestillation, entfernen oder einen Katalysator einsetzen, der entweder von vornherein an ein Polymeres, wie polymerisiertes Triphenylvinylphosphoniumfluorid, gebunden ist, oder der während der Reaktion an ein Polymeres gebunden wird. Vor der endgültigen Anwendung wird die Vernetzungsreaktion durch erneute Zugabe einer ausreichenden Katalysatormenge in Gang gesetzt.

Das Verfahren kann in Gegenwart oder Abwesenheit von organischen Lösungsmitteln durchgeführt werden, die gegebenenfalls auch einen Einfluß auf die Aktivität der Katalysatoren ausüben. Geeignete Lösungsmittel sind z.B. aromatische und aliphatische Kohlenwasserstoffe wie Toluol, die verschiedenen Xylole, Gemische aliphatischer und/oder aromatischer Kohlenwasserstoffe, Mineralölfraktionen, Ester, Äther, Alkohole oder dergleichen.

Alle Reaktionskomponenten können einzeln oder im Gemisch, sofern sie miteinander verträglich sind, eingesetzt werden.

Obwohl das Verfahren gewöhnlich unter Normaldruck abläuft, kann es in einzelnen Fällen erwünscht sein, auch unter erhöhtem Druck zu arbeiten, um die Härtungsgeschwindigkeit zu erhöhen.

Das gegenseitige Mengenverhältnis der Reaktionskomponenten A) und B), hängt von der Anzahl der ungesättigten Gruppen (I) der Verbindung A) und der Summe der aktiven H-Atome in Verbindung B) also einschließlich derjenigen in den Gruppen (II) (im folgenden kurz mit « aktive Doppelbindungen: aktive H-Atome" bezeichnet) ab. Bei der mehrstufigen Verfahrensführung kann es zur Herstellung der Vorprodukte innerhalb eines breiten Bereiches variiert werden. Zur Herstellung des vernetzten Endproduktes beträgt jedoch das genannte Verhältnis aktive Doppelbindungen: aktive H-Atome im allgemeinen etwa 2,4:0,8 bis 0,8:2,4, vorzugsweise etwa 2:1 bis 1:2, insbesondere (0,8 bis 1,2):1 bis 1:(0,8 bis 1,2). Für ein Mol Äthandiol-bisacrylat werden daher wegen der zwei aktiven H-Atome im Acetessigester z.B. nur etwa 0,8 bis 1,2 Mol Acetessigester angewendet. Will man hingegen nur ein H-Atom vom Acetessigester umsetzen, so kann man auch 2 × (0,8 bis 1,2) Mol Acetessigester einsetzen (Formel XII s. Formelblatt). Wie aus dieser Formel hervorgeht, enthält das Umsetzungsprodukt noch eine reaktionsfähige Gruppe (s. unterstrichenes H-Atom), so daß es entweder mit anderen Molekülen oder mit sich selbst weitervernetzen kann. Das Verhältnis aktive Doppelbindungen:aktive H-Atome ist dann 1:1. Andererseits ist es auch möglich, ein Gemisch der Komponenten A einerseits und/oder B andererseits mit jeweils unterschiedlicher Funktionalität einzusetzen, um den Vernetzungsgrad der Endprodukte zu steuern.

Das erfindungsgemäße Umsetzungsgemisch ist ein 2-Komponenten-System, das Topfzeiten zeigt, die je nach Auswahl der Verbindungen A) und B) sowie nach Art und Menge des Katalysators bzw. der Katalysatorkombination zwischen 5 min. und etwa 12 Stunden schwanken. Dadurch ist eine hohe Verarbeitungssicherheit gewährleistet. Aufgrund dieser vorteilhaften Eigenschaft des Produktes im Zusammenhang mit seiner raschen und einwandfreien Härtung bei Raumtemperatur oder erhöhter Temperatur und seiner chemischen Beständigkeit ist es hervorragend als Bindemittel für Überzüge geeignet.

Die 2-Komponenten-Systeme lassen sich als Überzüge auf vielerlei Unterlagen aufbringen, z.B. auf solche organischer oder anorganischer Natur, wie Holz, Holzfaserstoffe, z.B. als Holzversiegelung, Textilien natürlicher oder synthetischer Herkunft, Kunststoffe, Glas, Keramik, Baustoffe, wie Beton, Faserplatten, Kunststeine, insbesondere jedoch auf Metall. Die Überzüge können auch für Gegenstände und Geräte im Haushalt

und Gewerbe eingesetzt werden, z.B. Kühlgeräte, Waschmaschinen, Elektrogeräte, Fenster, Türen, Mögel oder dergleichen. Bevorzugt ist jedoch der Einsatz für Kraftfahrzeuge. Das Aufbringen kann durch Streichen, Sprühen, Tauchen oder elektrostatisch durchgeführt werden. Selbstverständlich können die 2-Komponenten-Systeme noch die üblichen Zusatzstoffe enthalten wie Farbstoffe, Pigmente, Füllstoffe, Weichmacher, Stabilisatoren, Verlaufmittel, neutralisierende Stoffe wie tertiäre Amine und Katalysatoren, die in den üblichen Mengen verwendet werden können. Diese Substanzen können den Einzelkomponenten und/oder der Gesamtmischung zugesetzt werden.

Als Farbstoffe bzw. Pigmente, die anorganischer oder organischer Natur sein können, werden beispielsweise genannt: Titandioxyd, Graphit, Ruß, Zinkchromat, Strontiumchromat, Bariumchromat, Bleichromat, Bleicyanamid, Bleisilikochromat, Calciummolybdat, Manganphosphat, Zinkoxyd, Cadmiumsulfid, Chromoxyd, Zinksulfid, Nickeltitangelb, Chromtitangelb, Eisenoxydrot, Eisenoxydschwarz, Ultramarinblau, Phthalocyaninkomplexe, Naptholrot oder dergleichen.

Geeignete Füllstoffe sind z.B. Talkum, Glimmer, Kaolin, Kreide, Quarzmehl, Asbestmehl, Schiefermehl, Bariumsulfat, verschiedene Kieselsäuren, Silikate oder dergleichen.

Für die Füller werden die üblichen Lösungsmittel verwendet beispielsweise aliphatische und aromatische Kohlenwasserstoffe, Äther, Ester, Glykoläther sowie deren Ester, Ketone, Chlorkohlenwasserstoffe, Terpenderivate, wie Toluol, Xylol, Äthyl- und Butylacetat, Äthylenglykolmonoäthyl- oder -butylätheracetat, Äthylenglykoldimethyläther, Diäthylenglykoldimethyläther, Cyclohexanon, Methyläthylketon, Aceton, Isophoron oder Mischungen davon.

Im Rahmen der Erfindung liegen daher auch 2-Komponenten-Systeme, die sich zur Herstellung von Überzügen, vorzugsweise Kraftfahrzeuglakken und insbesondere Autoreparaturlacken eignen. Hierbei ist die Xylolbeständigkeit und damit gleichzeitig eine gute Beständigkeit gegenüber Superbenzin vor allem von Interesse. Deshalb ist die erfindungsgemäße Verwendung der Umsetzungsprodukte besonders vorteilhaft. Da das 2-Komponenten-System nach einer bevorzugten Ausführungsform der Erfindung in verhältnismäßig kurzer Zeit bei Raumtemperatur selbst härten kann, ohne daß umweltbelastende Substanzen frei werden, ist seine Verwendung als Autoreparaturlack von erheblicher praktischer Bedeutung.

In den nachstehenden Vorschriften und Beispielen bedeuten % jeweils Gew.-% und T jeweils Gew.-Teile. Unter Vakuum wird jeweils das der Wasserstrahlpumpe verstanden.

## Beispiele

### I) Herstellung der Komponente A (Michael-Acceptor)

A 1) 400 T eines lösungsmittelfreien Acrylatharzes ®Macrynal SM 510 der Hoechst AG) (OH-Zahl 150) wurden mit 400 T Acrylsäure, 200 T Toluol, 3 T Hydrochinonmonomethyläther und 3 T p-Toluolsulfonsäure versetzt. Unter Durchleiten von Luft wurde bis zur Beendigung der $H_2O$-Entwicklung am Wasserabscheider erhitzt. Nach Abkühlen auf Raumtemperatur und Waschen mit Wasser wurde die überschüssige Acrylsäure entfernt. Die organische Phase wurde durch Destillation unter Vakuum vom Lösungsmittel befreit und auf einen Festkörpergehalt von 60% verdünnt. C = C-Äquivalentgewicht 864.

A 2) 510 T des unter A 1 genannten Acrylatharzes wurden mit 500 T Acrylsäuremethylester, 3 T Hydrochinonmonomethyläther und 6 T Dibutylzinnoxid versetzt. Binnen 30 h wurden über eine Vigreux-Kolonne 26T Methanol bei 80 bis 90°C Innentemperatur abdestilliert. Unter Vakuum wurde der überschüssige Acrylsäuremethylester abdestilliert. Der Rückstand wurde mit Xylol auf einen Festkörpergehalt von 60% verdünnt. C = C-Äquivalentgewicht 1083.

A 3) 1000 T eines glycidylgruppenhaltigen Acrylatharzes hergestellt aus Styrol, Glycidylmethacrylat und Dimethylmaleinat (Epoxidäquivalentgewicht 510) wurden in 660 T Xylol bei 70°C gelöst. Bei 70°C wurden 127 T Acrylsäure und 1 T Tetraäthylammoniumbromid zugesetzt. Unter Durchleiten von Luft wurde bei 80°C bis zur Säurezahl 1 nachgerührt. Die hellgelbe Lösung wurde mit 17 T Xylol verdünnt. Festkörpergehalt 62,5%, C = C-Äquivalentgewicht 1022.

A 4) 570 T der Harzlösung aus Beispiel A 3) wurden mit 0,1 T Dibutylzinndilaurat und 50 T n-Butylisocyanat versetzt. Es wurde bei 60°C 4 h lang nachgerührt (NCO-Wert 0,2%). Festkörpergehalt 65%; C = C-Äquivalentgewicht 1112.

A 5) 296 T Trimethylolpropantrisacrylat wurden in einem mit Rührer, Rückflußkühler und einer Gaseinleitung versehenen 1-l-Kolben vorgelegt und mit 200 T Isopropanol und 1,5 T Triäthylamin versetzt. Bei Raumtemperatur wurden 17 T Schwefelwasserstoff absorbiert. Es wurde eine weitere Stunde bei 30°C gerührt. Dann wurde die gebildete schwerere Produktphase als fast farblose Flüssigkeit abgetrennt. Festkörpergehalt 88%; C = C-Äquivalentgewicht 172.

A 6) In einem Dreihalskolben, versehen mit Rührer, Thermometer sowie Destillier-Kolonne mit absteigendem Kühler, wurden 657 T eines Melaminharzes vom Typ Hexamethoxymethylmelamin (Molekulargewicht 399), 1053 T 2-Hydroxyäthylacrylat, 3,3 T Hydrochinonmonomethyläther und 1,65 T Schwefelsäure vorgelegt. Im Vakuum wurde auf 75°C erwärmt und binnen 4 h auf 95°C erhitzt. Nach Abkühlung auf Zimmertemperatur wurde mit 10 T einer 10%igen methanolischen Kalilauge neutralisiert und filtriert. Man erhielt 141 T einer niedrigviskosen klaren Harzlösung. C = C-Äquivalentgewicht 175.

A 7) 220 T Isophorondiisocyanat wurden mit 1 T Dibutylzinndilaurat versetzt und auf 50°C erwärmt. Bei gleicher Temperatur wurden binnen einer Stunde 2 T Hydrochinonmonomethyläther, gelöst in 232 T Hydroxyäthylacrylat, zugetropft. Es wurde nachgerührt, bis der N = C = O-Gehalt kleiner als 0,5 war. Nach Zugabe von 7 T eines Glycidylesters einer gesättigten, in α-Stellung verzweigten $C_{9-11}$-Fettsäure (Epoxidäquivalentgewicht 260) und 0,5 T Chrom-III-octoat wurde 5 h lang bei 80°C nachgerührt und anschließend mit 115 T Xylol versetzt. Festkörpergehalt 80%; C = C-Äquivalentgewicht 289.

II) Herstellung der Komponente B (Michael-Donator)

B 1) in einem 2-l-Glaskolben, ausgestattet mit einer Destillationsbrücke, Rührer und Kontaktthermometer, wurden 312 T Neopentylglykol und 706 T Acetessigsäuremethylester unter $N_2$ als Schutzgas auf 130 bis 160°C erhitzt. Nach Beendigung der Abdestillation des Methanols (nach ca. 7 h) wurde auf 120°C gekühlt. Der überschüssige Acetessigsäuremethylester wurde im Vakuum abdestilliert. Es verblieben 819 T einer klaren hellen Flüssigkeit als Rückstand. C-H-Äquivalentgewicht 70.

B 2) In einer 4l-Apparatur analog B 1) wurden 670 T Trimethylolpropan und 1972 T Acetessigsäuremethylester binnen 5 h von 130 auf 180°C erhitzt. Nach beendeter Destillation befanden sich 465 T Methanol in der Vorlage. Nach Abkühlen auf 150°C wurde der niedrigsiedende Anteil im Vakuum abdestilliert. Es verblieben 1950 T einer farblosen Flüssigkeit als Rückstand. C-H-Äquivalentgewicht 64.

B 3) In der gleichen Apparatur wie nach B 1) wurden 335 T Trimethylolpropan und 2400 T Malonsäurediäthylester unter $N_2$- als Schutzgas auf 150 bis 170°C erhitzt. Nach beendeter Abdestillation des Äthanols wurde der überschüssige Malonsäurediäthylester im Vakuum bei 150 bis 160°C abdestilliert. Es verblieben als Rückstand 1201 T einer farblosen Flüssigkeit. C-H-Äquivalentgewicht 79.

B 4) In einer Apparatur wie in B 1) wurden 92 T Glycerin und 426 T 2,2,6-Trimethyl-4-oxo-4H-1,3-dioxin vorgelegt. Unter Rühren wurden binnen 5 h bei 180°C 160 T Aceton abdestilliert. Anschließend wurde der Rückstand bei 120°C im Vakuum von niedrigsiedenden Anteilen befreit. Es verblieben 348 T einer gelben niedrigviskosen Flüssigkeit. C-H-Äquivalentgewicht 58.

B 5) Unter Rühren wurden in einem 1-l-Dreihalskolben, der mit Rührer, Rückflußkühler und Kontaktthermometer versehen war, 300 T Acetessigsäureäthylester, 3 T Kalilauge und 4 T Hydrochinonmonomethyläther vorgelegt und auf 130°C erhitzt. Binnen einer Stunde wurden 198 T Butandioldiacrylat zudosiert. Nach weiteren 2 h betrug der Doppelbindungsanteil weniger als 0,2%. Anschließend wurde die Kalilauge mit methanolischer Salzsäure neutralisiert. Durch Anlegen von Hochvakuum wurden bei 100°C die niedrigsiedenden Anteile entfernt. Es verblieben 464 T einer hellgelben viskosen Flüssigkeit. C-H-Äquivalentgewicht 232.

B 6) 250 T eines Polyäthylenglykoldiamins (Aminzahl 243), gelöst in 166 T Diäthylenglykoldimethyläther, wurden bei -20°C zu einer Lösung von 190 T Diketen in 134 T Diäthylenglykoldimethyläther getropft. Nach 1½ h Rühren bei -28°C wurde auf + 10°C erwärmt und bis zu einer Aminzahl unter 1 nachgerührt. Nach Zugabe von 10 T Äthanol wurde für 40 min auf 90°C erhitzt. Anschließend wurde das Lösungsmittel im Vakuum abdestilliert. Der Rückstand wurde in 100 T Dimethoxyäthan aufgenommen. Man erhielt eine hellgelbe klare Lösung. Festkörpergehalt 81%; C-H-Äquivalentgewicht 122.

B 7) 134 T Trimethylolpropan und 327 T Cyanessigsäuremethylester wurden mit 4 T Titanacetylacetonat von 100 bis 180°C unter Abdestillieren von Methanol erhitzt. Nach 4 h wurde auf 140°C abgekühlt und der niedrigsiedende Anteil im Vakuum abdestilliert. Es verblieben 341 T einer gelben Flüssigkeit. C-H-Äquivalentgewicht 57.

B 8) 750 T des unter A 1) genannten Acrylatharzes (OH-Zahl 150) und 260 T Acetessigsäureäthylester wurden in einer Destillationsapparatur auf 150°C erhitzt. Innerhalb von 2 h wurde auf 170°C erhitzt. Nach beendeter Abdestillation des Äthanols wurde der niedrigsiedende Anteil nach Anlegen von Vakuum entfernt. Nach Abkühlen auf 100°C wurden 306 T Xylol zugegeben. Man erhielt eine klare hellgelbe Flüssigkeit. Festkörpergehalt 75%; CH-Äquivalentgewicht 310.

B 9) 510 T eines auf Basis Trimethylolpropan, Terephthalsäure, Adipinsäure und Neopentylglykol hergestellten Polyesters (OH-Zahl 110) wurden zusammen mit 130 T Acetessigsäureäthylester auf 140°C erhitzt. Binnen 3 h wurde unter fortlaufender Abdestillation flüchtiger Bestandteile auf 170°C erhitzt. Nach Abkühlen auf 140°C wurde der niedrigsiedende Anteil im Vakuum abdestilliert. Anschließend wurde mit

198 T Xylol verdünnt. Man erhielt eine hellgelbe klare Lösung. Festkörpergehalt 75%; C-H-Äquivalentgewicht 402.

B 10) 300 T des unter A 1 genannten OH-gruppenhaltigen Acrylatharzes wurden in 200 T Xylol bei 70°C gelöst. Nach Abkühlen auf Raumtemperatur wurden 0,025 T Dimethylaminopyridin zugesetzt. Innerhalb von 6 h wurden 60 T Diketen eingetropft. Nach weiteren 12 h war der Gehalt an freiem Diketen 0,2%. Festkörpergehalt 65%; C-H-Äquivalentgewicht 392.

B 11) 440 T Kokosölfettsäuremethylester (Verseifungszahl 255) wurden zusammen mit 268 T Trimethylolpropan und 1 T Butyltitanat in einer Destillationsapparatur auf 180°C erhitzt. Binnen 7 h wurde die Temperatur unter fortlaufender Abdestillation von Methanol langsam auf 250°C gesteigert. Nach Abdestillation von insgesamt 59 T Methanol wurde auf 100°C abgekühlt und mit 585 T Acetessigsäureäthylester versetzt. Nach Erwärmung auf 140°C wurde unter fortlaufender Destillation binnen 3 h auf 175°C erhitzt. Insgesamt wurden weitere 175 T Destillat erhalten. Nach Abkühlen auf 150°C wurde der niedrigsiedende Anteil im Vakuum entfernt. Es wurden 992 T einer viskosen hellgelben Flüssigkeit erhalten. C-H-Äquivalentgewicht 124.

B 12) 200 T Acetylaceton und 0,5 T Trimethylbenzylammoniumhydroxid wurden auf 40°C erwärmt. Binnen 2 h wurden 170 T Trimethylolpropantrisacrylat zugetropft. Anschließend wurde bis zu einem Doppelbindungsgehalt von unter 0,5% nachgerührt. Man erhielt eine hellgelbe viskose Flüssigkeit. C-H-Äquivalentgewicht 162.

III) Herstellung eines selbstvernetzenden Umsetzungsproduktes gemäß der Erfindung

C) (Beispiel 17) 924 T einer 70%igen Lösung eines durch radikalische Polymerisation von Styrol, Hydroxyäthylmethacrylat und Methylmethacrylat hergestellten Acrylatharzes (OH-Zahl 130) (Trägersubstanz) in Xylol wurden bei 40°C mit 258 T einer 70%igen Lösung eines mit Hydroxyäthylacrylat halbverkappten Toluylendiisocyanats (N = C = O-Gehalt 9,8%, C = C-Äquivalentgewicht 290) (Komponente A) in Xylol in Gegenwart von 5 T Dibutylzinndilaurat bis zu einem Isocyanat-Gehalt von weniger als 0,3% umgesetzt. Anschließend gab man 2 T Dimethylaminopyridin zu und tropfte bei Raumtemperatur unter Rühren 25 T Diketen (Komponente B) zu. Nach 24stündigem Rühren bei Raumtemperatur ließ sich kein freies Diketen mehr nachweisen. Festkörpergehalt 71%; C = C-Äquivalentgewicht 2022; C-H-Äquivalentgewicht 2020. Anstelle von Diketen kann auch die äquivalente Menge Acetessigester umgesetzt werden, wobei man ein Produkt mit denselben Kennzahlen erhält.

IV) Herstellung der Beschichtungen - Beispiele 1 bis 51

Beispiele 1 bis 51 gemäß der Erfindung sind in Tabellen 1 und 2 zusammengefaßt. Außerdem sind noch vier Vergleichsversuche V 1 bis 4 mit handelsüblichen Produkten ausgeführt.

Die in nachstehenden Tabellen 1 und 2 angegebenen Gewichtsmengen der Komponenten A und B wurden gemischt. Als Vergleichssubstanz diente ein aus 31 T Glycidylmethacrylat, 15 T Dimethylmaleinat und 54 T Styrol hergestelltes Copolymerisat. Als Härter für dieses System diente Diäthylentriamin. Im Fall der pigmentierten Überzüge wurde die dem gewünschten Pigmentierungsgrad entsprechende Menge an Titandioxid zugemischt und das Gemisch auf einer Perlmühle abgerieben. Das erhaltene Beschichtungsmaterial wurde als solches oder, falls erforderlich, nach Zumischung des angegebenen Katalysators mittels eines Aufziehrakels in einer Naßfilmstärke von 100 µm auf Glasplatten aufgebracht und bei Raumtemperatur bzw. bei 80°C (30 min) gehärtet.

Die in den nachstehenden Tabellen benutzten Abkürzungen bedeuten:

| | |
|---|---|
| DETA | Diäthylentriamin |
| HH | Heisshärtung = 30 min bei 80°C |
| RT | Raumtemperatur |
| TBAF | Tetrabutylammoniumfluorid |
| TMPSG | Trimethylolpropantrithioglykolsäureester |
| TMPTA | Trimethylolpropantriacrylat |
| TPTP | Tris-p-tolylphosphan |
| MDPP | Methyl-diphenylphosphan |
| PDOAP | Phenyl-di-o-anisylphosphan |
| DPOAP | Diphenyl-o-anisylphosphan |
| TPAP | Tris-p-anisylphosphan |
| MDPAP | Methyl-di-p-anisylphosphan |
| DPPDAPP | Diphenyl-p-dimethylamino-phenylphosphan |

| | |
|---|---|
| THMP | Trishydroxymethylphosphan |
| TCEP | Tris-2-cyanoethylphosphan |
| TDAMP | Tris-diethylaminomethylphosphan |
| TDAIP | Tris-dimethylamino-1,1-dimethylbenzyliminophosphoran |
| TBIP | Tributyl-1,1-dimethyl-benzyliminophosphoran |
| TPP | Triphenylphosphan |

V) Diskussion der Ergebnisse

Wie aus Tabelle 1 hervorgeht, zeigen die Produkte der Beispiele 1 bis 38 hohe chemische Beständigkeit und hohe Härte, d.h. sie sind vollständig vernetzt, obwohl die Härtung z.T. nur bei Raumtemperatur erfolgt ist. Andererseits weisen sie noch günstige Topfzeiten auf, vergleiche insbesondere Beispiele 6 bis 9,16-18, 22, 23, 27, 30. Die zuletzt genannten Beispiele zeigen, daß durch geeignete Auswahl der Katalysatoren eine Topfzeit im Bereich von 5 bis zu ca 20 h erzielt werden kann. Die Beispiele 11 bis 13 sowie 16, 19-21, 24,

Tabelle 1
Klarlackfilm (100 µ Nassfilm)

| Beispiel | Komponente A Typ | Komponente A Menge | Komponente B Typ | Komponente B Menge | Katalysator Typ | Katalysator Menge | Topfzeit (h) | Härtungstemperatur | Pendelhärte nach König (s) nach 1 Tg | Pendelhärte nach König (s) 10 Tg | Beständigkeit gegen (nach 10 Tagen) Xylol |
|---|---|---|---|---|---|---|---|---|---|---|---|
| 6 | A3 | 102 | B2 | 6,5 | TBAF | 0,6 | 5 | RT | 98 | 202 | > 24 h |
| 7 | TMPTA | 99 | B8 | 310 | TBAF | 4,0 | 6 | RT | 70 | 104 | 1 h |
| 8 | A6 | 88 | B8 | 310 | TBAF | 4,0 | 8 | RT | 80 | 123 | 1 h |
| 9 | A6 | 44 | B8 | 310 | TBAF | 3,5 | 11 | RT | 160 | 200 | 30 min |
| 12 | A3 | 30,7 | TMPSG | 3,6 | TBAF | 0,2 | 0,6 | HH | 191 | 200 | 1 h |
| 13 | A3 | 30,7 | DETA | 0,7 | – | – | 0,3 | HH | 151 | 160 | 30 min |
| 14 | – | | Typ C | 54 | TBAF | 0,3 | 0,4 | RT | 125 | 199 | 2 h |
| 15 | A3 | 102 | B2 | 6,5 | TPP | 0,7 | 28 | RT | 143 | 199 | > 24 h |
| 16 | A3 | 102 | B2 | 6,5 | TPTP | 0,4 | 12 | HH | 136 | 217 | > 24 h |
| 17 | A3 | 102 | B2 | 6,5 | MDPAP | 0,4 | 10 | HH | 139 | 216 | > 24 h |
| 18 | A3 | 102 | B2 | 6,5 | TPAP | 0,4 | 16 | HH | 125 | 210 | > 24 h |
| 21 | A3 | 102 | B2 | 6,5 | MDPP | 0,4 | 11 | HH | 124 | 189 | > 24 h |
| 22 | A3 | 102 | B2 | 6,5 | RDOAP | 0,4 | 15 | HH | 136 | 192 | > 24 h |
| 23 | A3 | 102 | B2 | 6,5 | DPOAP | 0,4 | 17 | HH | 133 | 196 | > 24 h |
| 24 | A3 | 102 | B2 | 6,5 | DPPDAPP | 0,4 | 4,5 | HH | 142 | 202 | > 24 h |
| 25 | A3 | 102 | B2 | 6,5 | THMP | 0,4 | 1,5 | RT | 141 | 200 | > 24 h |
| 26 | A3 | 102 | B2 | 6,5 | TCEP | 0,4 | 6 | HH | 122 | 201 | > 24 h |
| 27 | A3 | 102 | B2 | 6,5 | TDAMP | 0,4 | 16 | HH | 130 | 199 | > 24 h |
| 28 | A3 | 102 | B2 | 6,5 | TDAIP | 0,4 | 2,5 | HH | 140 | 202 | > 24 h |
| 29 | A3 | 102 | B2 | 6,5 | TBIP | 0,4 | 9 | RT | 140 | 198 | > 24 h |
| 30 | A3 | 102 | B3 | 8,0 | TBIP | 0,7 | 12 | HH | 98 | 176 | 14 h |
| 31 | A3 | 102 | B3 | 8,0 | DPPDAPP | 0,7 | 25 | HH | 95 | 172 | 10 h |
| 32 | A3 | 102 | B3 | 8,0 | TDAIP | 0,4 | 8 | HH | 101 | 182 | 12 h |
| 33 | A3 | 102 | B3 | 8,0 | MDPP | 0,7 | 1 | RT | 120 | 193 | > 24 h |
| 34 | A3 | 102 | B3 | 8,0 | MDPP | 0,4 | 24 | RT | 104 | 191 | > 24 h |

* Vergleich

Tabelle 1 (Fortsetzung)
Klarlackfilm (100 µ Nassfilm)

| Beispiel | Komponente A Typ | Komponente A Menge | Komponente B Typ | Komponente B Menge | Katalysator Typ | Katalysator Menge | Topfzeit (h) | Härtungstemperatur | Pendelhärte nach König (s) nach 1 Tg | Pendelhärte nach König (s) 10 Tg | Beständigkeit gegen (nach 10 Tagen) Xylol | |
|---|---|---|---|---|---|---|---|---|---|---|---|---|
| 38 | A3 | 102 | B3 | 8,0 | TBAF | 0,8 | 24 | HH | 92 | 179 | 10 h | |
| V1 (Vergleich) | | 45,8 | DETA | 2,1 | – | – | 24 h | RT | 65 | 117 | 10 min | 20 min |
| V2 (Vergleich) | | 45,8 | DETA | 2,1 | – | – | 24 h | HH | 132 | 142 | 15 min | 50 min |

Tabelle 2
Pigmentierte Beschichtungen (100 µ Nassfilm)

| Beispiel | Komponente A Typ | Komponente A Menge | Komponente B Typ | Komponente B Menge | Katalysator Typ | Katalysator Menge | $TiO_2$ | Topfzeit (h) | Härtungstemperatur | Pendelhärte nach König (s) nach 1 Tg | Pendelhärte nach König (s) 10 Tg | Beständigkeit gegen (nach 10 Tagen) Xylol | |
|---|---|---|---|---|---|---|---|---|---|---|---|---|---|
| 41 | A6 | 88 | B2 | 32 | TBAF | 1,3 | 96,5 | 35 | RT | 145 | 190 | > 24 h | |
| 42 | TMPTA | 49 | B2 | 32 | {TBAF<br>BTAH* | 0,3<br>0,3} | 65,2 | 30 | RT | 127 | 174 | > 24 h | |
| 44 | {A6<br>TMPTA | 44<br>25} | B2 | 32 | TBAF | 1 | 81,2 | 35 | RT | 145 | 175 | 6 h | |
| 48 | A3 | 255,5 | B2 | 32 | TBAF | 1,4 | 154 | 30 | RT | 97 | 174 | > 24 h | |
| 49 | A3 | 128,0 | B8 | 39 | TBAF | 0,8 | 87 | 40 | RT | 77 | 162 | 8 h | |
| 50 | A3 | 30,7 | TMPSG | 3,6 | TBAF | 0,4 | 18,2 | 35 | HH | 180 | 182 | 1 h | |
| 51 | A3 | 30,7 | DETA | 0,7 | – | – | 15,9 | 25 | HH | 140 | 157 | 30 min | |
| V3 (Vergleich) | | 45,8 | DETA | 2,1 | – | – | 38,3 | 16 h | RT | 55 | 117 | 12 min | 25 min |
| V4 (Vergleich) | | 45,8 | DETA | 2,1 | – | – | 38,3 | 16 h | HH | 138 | 147 | 20 min | 50 min |

* Vergleich

28, 33 bis 37 lassen erkennen, daß bei der forcierten Trocknung (Heißhärtung 30 min/80°C) bereits nach einem Tag hohe Härtewerte erhalten werden. Die starken Basen, die in den Vergleichsversuchen 1 bis 5 sowie 10 und 11 (TBAH und BTAH) eingesetzt wurden, führen jedoch, wie andere Vergleichsversuche zeigen, zu verfärbten Überzügen.

Tabelle 2 zeigt im Vergleich zu Tabelle 1, daß die Topfzeiten der pigmentierten Systeme etwas kürzer sind, daß sie aber trotzdem für eine sichere Verarbeitung ausreichen. Wie aus den Beispielen 39 bis 49 hervorgeht, härten die Filme schon bei Raumtemperatur vollständig aus. Die chemische Beständigkeit der Produkte gemäß Tabelle 2 ist im Durchschnitt höher als die der unpigmentierten Systeme von Tabelle 1.

Die Beispiele 12, 13, 50 und 51 zeigen, daß auch die Amin- und Mercaptoverbindungen als Komponente B eine vollständige Aushärtung und gute chemische Beständigkeit der Produkte ergeben, wobei im Fall der Beispiele 13 und 51 sogar ohne Katalysator gearbeitet wird. Die physikalischen Werte der Vergleichsversuche V1 bis V 4 lassen eine geringe Xylolbeständigkeit erkennen und auch einen wenig ausgeprägten Härtungsgrad. Auch hier liefert der starke Katalysator BTAH in den weiteren Vergleichsversuchen 39, 40, 42, 43 und 45 bis 47 gemäß anderen Untersuchungen Überzüge mit schlechteren Farbzahlen. Die erfindungsgemäßen Produkte sind daher den Vergleichsproben überlegen.

## Formelblatt

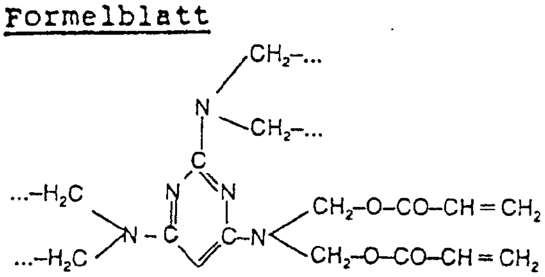

(III)

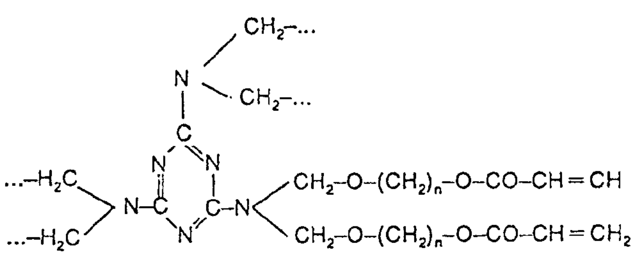

(IV)

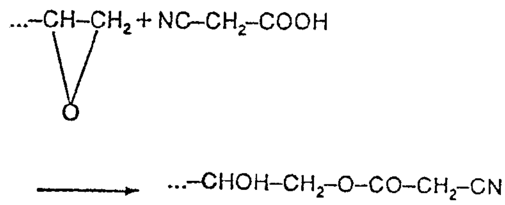

(V)

$$CH_3\text{-}CO\text{-}CH_2\text{-}CO\text{-}NH\text{-}(CH_2)_n\text{-}NH\text{-}CO\text{-}CH_2\text{-}CO\text{-}CH_3$$

$n = 2\text{-}10$ (VI)

(Alkyl 0)$_2$ PO-$CH_2$-COOR (VII)

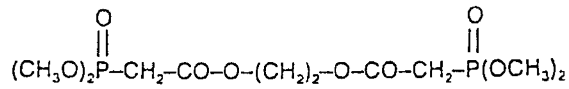

(VIII)

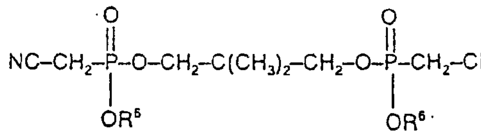

(IX)

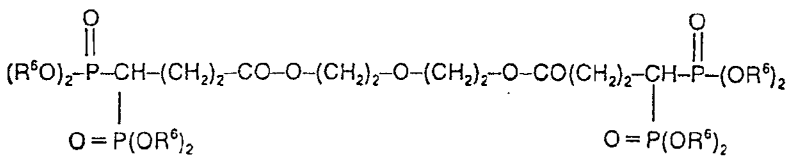

$R^6 = C_{1-5}$–Alkyl

(X)

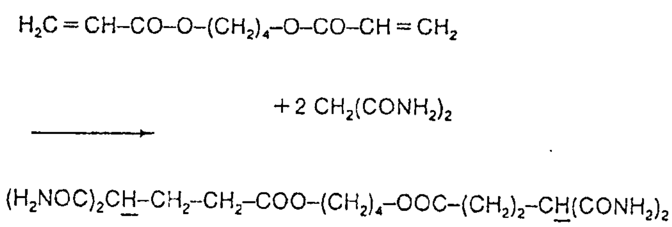


(XI)

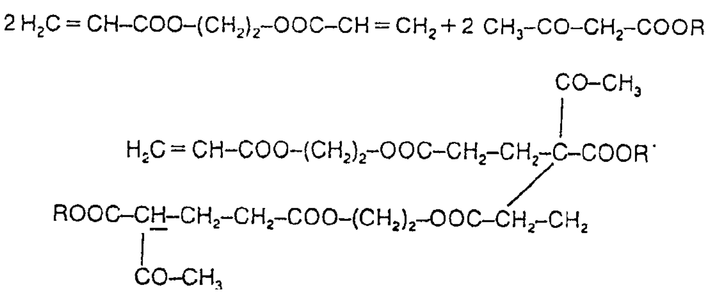

(XII)

## Patentansprüche

1. 2-Komponentenlack bestehend aus

   A) Verbindungen mit mindestens zwei $R^1R^2C = CR^3$-X-Gruppen (I),

   B) Verbindungen, die

   a) mindestens zwei aktive H-Atome oder

   b) mindestens zwei Gruppen mit aktiven H-Atomen des Typs -AH- (II) oder

   c) mindestens ein aktives H-Atom und mindestens eine Gruppe des Typs (II) enthalten bzw. die entsprechende Anzahl dieser Gruppe (II) bilden,

   worin in Formel (I) bedeuten

   X = -CO-, das entweder direkt oder über den Rest eines mehrwertigen Alkohols oder eines Amins an eine weitere $R^1R^2C = CR^3$-Gruppe gebunden ist,

   $R^1$ = Wasserstoff oder ein Kohlenwasserstoff-Rest mit 1 bis 10 C-Atomen,

   $R^2$ = Wasserstoff, ein Kohlenwasserstoff-Rest mit 1 bis 10 C-Atomen, eine Estergruppe mit dem Rest $R^{14}$ eines einwertigen Alkohols mit bis zu 12 C-Atomen, -CN, $-NO_2$, eine CO-$NHR^1$- oder eine

CO-$R^1$-Gruppe,
$R^3 = R^2$ und hiermit gleich oder verschieden ist, und in Formel (II)
-AH- für eine der Gruppierungen

$$-\overset{|}{C}H-$$

oder -SH steht, und

C) einem Katalysator aus der Gruppe Halogenide von quartären Ammoniumverbindungen, allein oder im Gemisch mit Kieselsäurealkylestern, oder organischen Phosphoniumsalzen mit 1 bis 20 C-Atomen im Alkylrest und/oder Arylrest sowie Phosphanen, für sich oder in Kombination mit üblichen Zusatzstoffen.

**2.** 2-Komponentenlack nach Anspruch 1, dadurch gekennzeichnet, daß der Katalysator C) ein Fluorid von quartären Ammoniumverbindungen oder ein Phosphan ist.

**3.** 2-Komponentenlack nach Anspruch 1, dadurch gekennzeichnet, daß der Katalysator C) ein Phosphan der allgemeinen Formel $P(CH_2\text{-}Y)_3$, in der Y gleich oder verschieden ist und den Rest -OH, -$CH_2CN$ oder -$N(Z)_2$ bedeutet, wobei Z ein Alkylrest mit 1 bis 5 C-Atomen ist, oder ein tertiäres Phosphan der allgemeinen Formel $P(R^4\ R^5\ R^6)$, in der Reste $R^4$, $R^5$ und $R^6$ einen Alkylrest mit 1 bis 12 C-Atomen oder einen Phenylrest, unsubstituiert oder substituiert mit mindestens einer Alkyl-, Alkoxy- oder Dialkylaminogruppe mit jeweils 1 bis 4 C-Atomen im Alkylrest bedeuten, $R^4$, $R^5$ und $R^6$ gleich oder verschieden sind, wobei jedoch mindestens einer der Reste einen Phenylrest darstellt, oder ein Aminophosphoran der allgemeinen Formel $(R^7,R^8,R^9)P = N\text{-}C(R^{10},R^{11},R^{12})$, in der $R^7$, $R^8$, $R^9$ gleich oder verschieden sind und einen Alkylrest mit 1 bis 12 C-Atomen oder einen Phenylrest, unsubstituiert oder substituiert mit mindestens einer Alkyl-, Alkoxy- oder Dialkylaminogruppe mit jeweils 1 bis 4 C-Atomen im Alkylrest bedeuten und $R^{10}$, $R^{11}$, $R^{12}$ gleich oder verschieden sind und einen Alkylrest mit 1 bis 5 C-Atomen oder einen Phenylrest darstellt, ist.

**4.** 2-Komponentenlack nach Anspruch 1, dadurch gekennzeichnet, daß der Katalysator C) Trishydroxymethylphosphan, Trisdimethylaminomethylphosphan, Tris-p-anisylphospan, Methyl-diphenylphosphan, Methyl-di-p-anisylphosphan oder $\alpha$, $\alpha$-Dimethylbenzyliminotributylphosphoran ist.

**5.** 2-Komponentenlack nach Anspruch 1, dadurch gekennzeichnet, daß in der Verbindung A) der Rest $R^1R^2C = CR^3\text{-}X$ (I) von der Acrylsäure abgeleitet ist und die Verbindung B) esterartig gebundene Malonsäureanteile enthält.

**6.** 2-Komponentenlack nach Anspruch 1, dadurch gekennzeichnet, daß der Rest $R^1R^2C = CR^3\text{-}X$ (I), worin X -CO- bedeutet und $R^3$ Wasserstoff oder Alkyl mit 1 bis 8 C-Atomen bedeuten, von einer ein- oder mehrfach ungesättigten, höchstens zweibasischen Carbonsäure mit 2 bis 10, vorzugsweise 3 bis 6 C-Atomen, vorzugsweise Acrylsäure, Methacrylsäure und/oder Maleinsäure abgeleitet ist.

**7.** 2-Komponentenlack nach Anspruch 1, dadurch gekennzeichnet, daß in den Verbindungen A) die Gruppen (I) an den Rest eines Polyols oder Polyamins, vorzugsweise eines Oligomeren und/oder Polymeren gebunden sind.

**8.** 2-Komponentenlack nach Anspruch 1, dadurch gekennzeichnet, daß die Verbindung A) von einem Polyester, Acrylharz, Epoxidharz, jeweils OH-Gruppen enthaltend, und/oder Aminharz abgeleitet ist.

**9.** 2-Komponentenlack nach Anspruch 1, dadurch gekennzeichnet, daß sich die Gruppierung -AH- (II) der Verbindung B)
aa) für die Bedeutung

$$-\overset{|}{C}H-$$

von einer Verbindung mit der Gruppierung -CO-CH$R^1$-CO-, NC-CH$R^1$-CO-, NC-$CH_2$-CN, =PO-CH$R^1$-CO-, =CH$R^1$-CN, =PO-CH$R^1$-PO=, -CO-CH$R^1$-$NO_2$,
bb) für die Bedeutung -SH von einem Thioalkoholsäureester, -amid und/oder einem Mercaptan ableitet.

**10.** 2-Komponentenlack nach Anspruch 1, dadurch gekennzeichnet, daß sich die Verbindung B) von einer

mindestens zweiwertigen Verbindung der Gruppe Polyole, Polyamine und/oder Polymercaptane ableitet.

**11.** 2-Komponentenlack nach Anspruch 1, dadurch gekennzeichnet, daß er sich auf einem Substrat, insbesondere einem Metall, vorzugsweise in dünner Schicht befindet.

## Claims

**1.** A 2-component lacquer composed of

A) compounds containing at least two $R^1R^2C=CR^3$-X groups (I) and

B) compounds containing

a) at least two active H atoms or

b) at least two groups having active H atoms of the type -AH- (II) or

c) at least one active H atom and at least one group of the type (II) or forming the corresponding number of groups (II),

whereby, in formula (I),

X denotes -CO- which is attached to a further $R^1R^2C=CR^3$ group, either directly or via the radical of a polyhydric alcohol or of an amine,

$R^1$ denotes hydrogen or a hydrocarbon radical having 1 to 10 carbon atoms,

$R^2$ denotes hydrogen, a hydrocarbon radical having 1 to 10 carbon atoms, an ester group containing the radical $R^{14}$ of a monohydric alcohol having up to 12 carbon atoms, -CN, $-NO_2$ or a CO-NHR$^1$ or CO-R$^1$ group,

$R^3$ has the same meaning as $R^2$ and is identical to or different from the latter,

and whereby, in formula (II),

-AH denotes one of the groupings

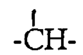

or -SH, and

C) a catalyst selected from the group including halides of quaternary ammonium compounds, either alone or in admixture with alkyl silicates, or organic phosphonium salts having 1 to 20 carbon atoms in their alkyl radical and/or aryl radical, and furthermore phosphanes, either alone or in combination with conventional additives.

**2.** The 2-component lacquer as claimed in claim 1, characterized in that the catalyst C) is a fluoride of quaternary ammonium compounds or a phosphane.

**3.** The 2-component lacquer as claimed in claim 1, characterized in that the catalyst C) is

- a phosphane of the formula $P(CH_2\text{-}Y)_3$, in which the radicals Y are identical to or different from one another and denote the radical -OH, $-CH_2CN$ or $N(Z)_2$, Z denoting an alkyl radical having 1 to 5 carbon atoms, or
- a tertiary phosphane of the formula $P(R^4R^5R^6)$, in which the radicals $R^4$, $R^5$ and $R^6$ denote an alkyl radical having 1 to 12 carbon atoms or a phenyl radical, unsubstituted or substituted by at least one alkyl, alkoxy or dialkylamino group, each of which has 1 to 4 carbon atoms in the alkyl radical, and $R^4$, $R^5$ and $R^6$ are identical to or different from one another, but at least one of these radicals represents a phenyl radical, or
- an aminophosphorane of the formula $(R^7R^8R^9)P=N-C(R^{10}R^{11}R^{12})$, in which $R^7$, $R^8$ and $R^9$ are identical to or different from one another and denote an alkyl radical having 1 to 12 carbon atoms or a phenyl radical, unsubstituted or substituted by at least one alkyl, alkoxy or dialkylamino group each of which has 1 to 4 carbon atoms in the alkyl radical, and $R^{10}$, $R^{11}$ and $R^{12}$ are identical to or different from one another and each represent an alkyl radical having 1 to 5 carbon atoms or a phenyl radical.

**4.** The 2-component lacquer as claimed in claim 1, characterized in that the catalyst C) is trishydroxymethyl phosphane, trisdimethylaminomethyl phosphane, tris-p-anisyl phosphane, methyl-diphenyl phosphane, methyl-di-p-anisyl phosphane or $\alpha,\alpha$-dimethylbenzylimino-tributyl phosphorane.

**5.** The 2-component lacquer as claimed in claim 1, characterized in that, in compound A), the radical $R^1R^2C=CR^3X$ (I) is derived from acrylic acid and in that compound B) contains malonic acid moieties attached by an ester-type linkage.

6. The 2-component lacquer as claimed in claim 1 characterized in that the radical $R^1R^2C=CR^3$-X (I), in which X denotes -CO- and $R^3$ denotes hydrogen or alkyl having 1 to 8 carbon atoms, is derived from a mono-unsaturated or polyunsaturated, at most dibasic carboxylic acid having 2 to 10, preferably 3 to 6, carbon atoms, preferably from acrylic acid, methacrylic acid and/or maleic acid.

7. The 2-component lacquer as claimed in claim 1, characterized in that, in compounds A), the groups (I) are attached to the radical of a polyol or polyamine, preferably of an oligomer and/or polymer.

8. The 2-component lacquer as claimed in claim 1, characterized in that the compound A) is derived from a polyester, an acrylic resin, an epoxide resin, in each case containing OH groups, and/or from an amine resin.

9. The 2-component lacquer as claimed in claim 1, characterized in that the grouping -AH (II) of compound B) is derived from
   aa) a compound having the grouping $-CO-CHR^1-CO-$, $NC-CHR^1-CO-$, $NC-CH_2-CN$, $=POCHR^1-CO-$, $=CHR^1-CN$, $=PO-CHR^1-PO=$ or $-CO-CHR^1-NO_2$ when it denotes -CH-, and from
   bb) a thioalcohol acid ester, thioalcohol acid amide and/or a mercaptan when it denotes -SH.

10. The 2-component lacquer as claimed in claim 1, characterized in that compound B) is derived from an at least divalent compound selected from the group including polyols, polyamines and/or polymercaptans.

11. The 2-component lacquer as claimed in claim 1, characterized in that it is present on a substrate, in particular a metal, preferably in the form of a thin coat.

## Revendications

1. Vernis à deux composants, formé
   A) de composés comportant au moins deux groupes $R^1R^2C=CR^3$-X- (I),
   B) de composés ayant
      a) au moins deux atomes de H actif, ou bien
      b) au moins deux groupes à atomes de H actif du type -AH (II), ou bien
      c) au moins un atome de H actif et au moins un groupe du type (II) ou formant le nombre correspondant de ces groupes (II), et, dans la formule (I)
   X représente -CO-, qui est lié, directement ou par l'intermédiaire du reste d'un polyalcool ou d'une amine, à un autre groupe $R^1R^2C=CR^3$-,
   $R^1$ représente un atome d'hydrogène ou un reste d'hydrocarbure comportant 1 à 10 atomes de carbone,
   $R^2$ représente un atome d'hydrogène, un reste d'hydrocarbure comportant 1 à 10 atomes de C, un groupe ester contenant le reste $R^{14}$ d'un monoalcool ayant jusqu'à 12 atomes de carbone, un groupe -CN, un groupe $-NO_2$, un groupe $-CONHR^1$ ou un groupe $-CO-R^1$, et
   $R^3$ a le même sens que $R^2$, les deux pouvant être identiques ou différents l'un de l'autre, et -AH- désigne un groupe -CH- ou -SH-, et
   C) d'un catalyseur pris parmi des halogénures de composés d'ammonium quaternaires, seul ou avec des silicates d'alkyles ou des sels de phosphonium organiques ayant de 1 à 20 atomes de carbone par alkyle et/ou aryle et des phosphanes, éventuellement avec des additifs courants.

2. Vernis selon la revendication 1, caractérisé en ce que le catalyseur C) est un fluorure d'un composé d'ammonium quaternaire ou un phosphane.

3. Vernis selon la revendication 1, caractérisé en ce que le catalyseur C) est un phosphane de formule générale $P(CH_2-Y)_3$, dans laquelle les Y sont identiques ou différents et représentent chacun un reste -OH, $-CH_2CN$ ou $-N(Z)_2$, Z représentant un reste alkyle ayant 1 à 5 atomes de carbone, ou bien un phosphane tertiaire de formule générale $P(R^4, R^5, R^6)$, dans laquelle les restes $R^4$, $R^5$ et $R^6$ représentent chacun un reste alkyle ayant 1 à 12 atomes de carbone ou un reste phényle, non substitué ou portant comme substituant au moins un groupe alkyle, alcoxy ou dialkylamino ayant chacun 1 à 4 atomes de carbone dans le reste alkyle, $R^4$, $R^5$ et $R^6$ sont identiques ou différents, mais l'un au moins de restes représente un reste

phényle, ou encore un aminophosphorane de formule générale ($R^7$, $R^8$, $R^9$)P=N-C($R^{10}$, $R^{11}$, $R^{12}$), dans laquelle les symboles $R^7$, $R^8$ et $R^9$ représentent des restes identiques ou différents et représentent chacun un reste alkyle ayant 1 à 12 atomes de carbone ou un reste phényle, non substitué ou substitué par au moins un groupe alkyle, alcoxy ou dialkylamino ayant chacun 1 à 4 atomes de carbone dans le reste alkyle, et $R^{10}$, $R^{11}$ et $R^{12}$ sont identiques ou différents et représentent chacun un reste alkyle ayant 1 à 5 atomes de carbone ou un reste phényle.

**4.** Vernis selon la revendication 1, caractérisé en ce que le catalyseur est du trishydroxyméthylphosphane, du trisdiméthylaminométhylphosphane, du méthyldiphénylphosphane, du méthyl-déphénylphosphane, du méthyl-di-p-anisylphosphane ou de l'$\alpha$,$\alpha$-diméthylbenzyliminotributylphosphorane.

**5.** Vernis selon la revendication 1, caractérisé en ce que, dans le composé A), le reste $R^1R^2C=CR^3$-X (I) dérive de l'acide acrylique, et le composé B) comporte des parties d'acide malonique lié en esters.

**6.** Vernis selon la revendication 1, caractérisé en ce que le reste $R^1R^2=CR^3$-X (I), dans lequel X représente -CO- et $R^3$ représente un atome d'hydrogène ou un groupe alkyle ayant 1 à 8 atomes de carbone, dérive d'un acide carboxylique insaturé une ou plusieurs fois, et qui est au maximum un diacide, comportant 2 à 10, de préférence 3 à 6, atomes de carbone, avantageusement l'acide acrylique, l'acide méthacrylique et/ou l'acide maléique.

**7.** Vernis selon la revendication 1, caractérisé en ce que, dans les composés A), les groupes (I) sont fixés sur le reste d'un polyol ou d'une polyamine, avantageusement d'un oligomère et/ou d'un polymère.

**8.** Vernis selon la revendication 1, caractérisé en ce que le composé A) dérive d'un polyester, d'une résine acrylique, d'une résine époxyde, avec chacun des groupes OH, et/ou dérive d'une résine d'amine.

**9.** Vernis selon la revendication 1, caractérisé en ce que le groupement -AH (II) du composé B) dérive

aa) . quand il représente

$$-\overset{|}{C}H-,$$

d'un composé comportant le groupe -CO-$CHR^1$-CO-, NC-$CHR^1$-CO-, NC-$CH_2$-CN, =PO-$CHR^1$-CO-, =$CHR^1$-CN, =PO-$CHR^1$-PO=, -CO-$CHR^1$-$NO_2$, et

bb). quand il représente -SH, d'un ester ou amide d'acide thioalcoolique et/ou d'un mercaptan.

**10.** Vernis selon la revendication 1, caractérisé en ce que le composé B) dérive d'un composé au moins divalent choisi parmi des polyols, des polyamines et/ou des polymercaptans.

**11.** Vernis selon la revendication 1, caractérisé en ce qu'il se trouve, avantageusement en couche mince, sur un substrat, en particulier sur un métal.